# EUROPEAN PATENT APPLICATION

(11) **EP 1 618 847 A2**
(43) Date of publication of application: **25.01.2006**
(21) Application number: 05106273.5
(22) Date of filing: 08.07.2005
(51) Int. Cl.: A61B 10/00

(54) **Tissue homogenizer device and method**

(30) Priority: 21.07.2004 US 895869
(71) Applicant: IDEXX LABORATORIES, INC., Westbrook, MA 04092 (US)
(72) Inventor: WONG, Cai'ne Woo, Buxton, MA 04093 (US); BARSKI, Stanislaw, Limerick. MA 04048 (US); LIBBY, Tracey Lynn Hessel, North Yarmouth, MA 04097 (US); TONELLI, Quentin Joseph, Portland, MA 04103 (US)
(74) Representative: Grey, Ian Michael

(57) **Abstract**

Tissue homogenizer devices for testing a sample of blood and/or tissue are provided. A device may include an outer cylinder (102); an inner cylinder (104) slidably disposed within a lumen of the outer cylinder and defining a chamber (124) therewithin; and a seal (118) extending across an open distal end of the inner cylinder. The device may further include a piston (130) extending proximally from a closed proximal end of the inner cylinder and through an open proximal end of the outer cylinder. Methods of using the devices disclosed herein are also provided.

## Description

The present disclosure relates to tissue homogenizing devices and methods of use thereof.

Biological samples, such at tissue samples (i.e. brain, lung, kidney etc...) and fluid samples (i.e. blood, plasma, urine, milk etc...) are well known sources of information regarding the condition or health of the host. For many assays involving such samples, particularly tissue samples, it is advantageous to break up or otherwise homogenize the sample to gain proper access to constituents contained therein.

Accordingly, a continuing need exists for an improved device for collection, separating and distributing tissue and blood samples for examination.

A need also exists for a method of using the improved device for collecting, and easily homogenizing tissue samples.

The present disclosure relates to a tissue homogenizer device for testing a sample of blood and/or tissue. The device includes an outer cylinder including an open distal end, an open proximal end, and defining a lumen therethrough; an inner cylinder slidably disposed within the lumen of the outer cylinder, the inner cylinder including an open distal end, a closed proximal end, and defining a chamber therewithin; and a seal extending across the open distal end of the inner cylinder. The device further includes a piston having a piston rod extending proximally from the closed proximal end of the inner cylinder and through the open proximal end of the outer cylinder.

Axial displacement of the piston axially displaces the inner cylinder relative to the outer cylinder, the device including a first position in which the distal end of the inner cylinder is in close proximity to the open distal end of the outer cylinder, and a second position in which the distal end of the inner cylinder is spaced a distance from the open distal end of the outer cylinder.

Desirably, when the device is in the first position the proximal end of the inner cylinder is spaced a distance from the proximal end of the outer cylinder, and when the device is in the second position the proximal end of the inner cylinder is in close proximity with the proximal end of the outer cylinder.

The device further includes particulate in the chamber of the inner cylinder. The particulate may include pellets, granules, shots, BBs and/or aggregate. The piston rod desirably includes a region of reduced strength formed along the length thereof.

The device further includes a cap removably connectable to the open distal end of the outer cylinder. The cap includes at least one rod extending therefrom for penetrating the seal of the inner cylinder when the cap is secured to the distal end of the outer cylinder. The cap desirably includes an engagement element for securing the cap to a complementary engagement element provided at the distal end of the outer cylinder.

The device further includes a dispensing cap removably connectable to the open distal end of the outer cylinder. The dispensing cap includes a lumen extending therethrough. The dispensing cap further includes an engagement element for securing the cap to a complementary engagement element provided at the distal end of the outer cylinder. The dispensing cap may further include a filter extending across the lumen thereof.

The device may further include a septum cap removably connectable to the open distal end of the outer cylinder. The septum cap includes an annular outer wall, an annular inner wall defining a passage and a septum seal extending across the passage. The septum seal is desirably made from rubber. The device may further include a filter slidably disposed in the chamber of the inner cylinder.

In one aspect of the present disclosure, the proximal end of the outer cylinder may include a hub operatively associated therewith through which the piston rod extends.

Additionally, the proximal end of the inner cylinder may include a hub operatively associated therewith to which the piston rod is secured.

The piston rod desirably slidably extends through an opening formed in the closed proximal end of the inner cylinder. The piston includes a head operatively connected to a distal end of the piston rod. The head desirably includes at least one aperture formed therethrough.

The piston rod includes a region of reduced strength formed along the length thereof enabling the piston rod to be separated from the head. The piston rod reciprocatingly drives the head axially through the chamber of the inner cylinder. A filter may be provided which extends across the opening formed in the proximal end of the inner cylinder.

The open proximal end of the outer cylinder may include a flange formed therearound. Desirably, the piston rod includes a series of indicia along the length thereof, wherein the indicia indicates a quantity of a sample drawn into the chamber and/or indicates a quantity of a sample expressed from the chamber.

According to another embodiment, the tissue homogenizer device may include a cylinder including an open distal end, a closed proximal end, and defining a chamber therewithin; a seal extending across the chamber to define a closed proximal reservoir and an open distal reservoir; particulate disposed in the proximal reservoir; and a cap operatively securable to the distal end of the cylinder. The cap desirably includes an end wall defining an opening therein; a seal extending across the opening formed in the end wall of the cap; and at least one puncturing element extending from an inner surface of the end wall of the cap, wherein the puncturing element is configured to penetrate the seal when the cap is secured to the distal end of the cylinder.

The distal end of the cylinder may include a cutting edge, wherein the cutting edge is desirably serrated. The cap may include an engagement element for engaging a complementary engagement element provided on the cylinder.

According to another aspect of the present disclosure, a method of examining a tissue or blood sample is provided. The method includes the steps of providing a tissue homogenizing device. The device includes an outer cylinder having an open distal end, an open proximal end, and defining a lumen therethrough; an inner cylinder slidably disposed within the outer cylinder, the inner cylinder having an open distal end, a closed proximal end, and defining a chamber therewithin; a seal extending across the chamber of the inner cylinder; particulate disposed within the chamber of the inner cylinder; a piston having a piston rod extending through the open proximal end of the outer cylinder and operatively associated with the closed proximal end of the inner cylinder; and a cap securable to the distal end of the outer cylinder. The device has a first position in which the proximal end of the inner cylinder is spaced a distance from the proximal end of the outer cylinder, wherein the distal end of the inner cylinder does not extend beyond the distal end of the outer cylinder, and at least one second position in which the proximal end of the inner cylinder is in close proximity to the proximal end of the outer cylinder.

The method further includes the steps of inserting the distal ends of the outer cylinder and the inner cylinder into a quantity of a sample; at least one of withdrawing the piston to proximally displace the inner cylinder relative to the outer cylinder to evacuate the lumen of the outer cylinder and draw in a test sample of the quantity of sample, and urging at least the distal end of the outer cylinder into the quantity of the sample; and securing the cap onto the distal end of the outer cylinder.

The cap desirably includes a piercing element extending therefrom such that when the cap is secured to the distal end of the outer cylinder the piercing element penetrates the seal. The method further includes the step of separating the piston rod from the inner cylinder. The piston rod desirably includes a series of indicia along the length thereof indicating a volume of test sample acquired in the lumen of the outer cylinder.

The method further includes the step of agitating the device following separation of the piston from the inner cylinder. The method further includes the step of replacing the cap with a dispensing cap following agitation of the device. The dispensing cap desirably defines a lumen extending therethrough and includes a filter extending across the lumen thereof. The method further includes the step of urging the inner cylinder in a distal direction relative to the outer cylinder to force the test sample through the filter and out through the lumen of the dispensing cap.

Desirably, the piston rod slidably extends through an opening formed in the proximal end of the inner cylinder. The piston includes a head provided on the distal end of the piston rod and disposed within the chamber of the inner cylinder. The piston rod has a first position in which the head is in close proximity to the proximal end of the inner cylinder and at least one second position in which the head is spaced a distance from the proximal end of the inner cylinder. Desirably, the piston rod is separable from the head.

The device may further include a septum seal extending across the opening formed in the proximal end of the outer cylinder. The method further includes the step of urging the head from the first position to at least one second position, inserting a pipette into the chamber of the outer cylinder through the opening in the proximal end of the cylinder, and withdrawing the test sample from the device. Desirably, the head includes at least one aperture formed therethrough.

According to another aspect, the method further includes the step of replacing the cap with a septum cap. The septum cap desirably defines a passage therethrough and which includes a seal extending across the passage. The device may further include a filter slidably disposed within the chamber of the inner cylinder.

Further features of the above embodiments and methods will become more readily apparent to those skilled in the art from the following detailed description of the apparatus taken in conjunction with the drawings.

By way of example only, preferred embodiments of the disclosure will be described with reference to the accompanying drawings, in which:
Figure 1 is a side cross sectional elevational view of a universal tissue homogenizing device, in accordance with one illustrative embodiment of the present disclosure, illustrating a stage of use thereof;
Figure 2 is a side elevational view of the tissue homogenizing device of Figure 1 illustrating another stage of use thereof;
Figure 3 is a side elevational view of the tissue homogenizing device of Figures 1 and 2 illustrating yet another stage of use thereof;
Figure 4 is a side elevational view of the tissue homogenizing device of Figures 1-3 as configured for manual use in still another stage of use thereof;
Figure 5 is a side elevational view of the tissue homogenizing device of Figures 1-3 as configured for automated use in still a further stage of use thereof;
Figure 6 is a side cross sectional elevational view of a universal tissue homogenizing device, in accordance with another illustrative embodiment of the present disclosure, illustrating a stage of use thereof;
Figure 7 is a side elevational view of the tissue homogenizing device of Figure 6 illustrating another stage of use thereof;
Figure 8 is a side cross-sectional elevational view of the tissue homogenizing device of Figures 6 and 7 illustrating yet another stage of use thereof;
Figure 9 is a side cross-sectional elevational view of the tissue homogenizing device of Figures 6-8 as configured for manual use in still another stage of use thereof;
Figure 10A is a cross-sectional side elevational view of the tissue homogenizing device of Figures 6-9, further including a piston having a tip configured and adapted for filtering;
Figure 10B is a plan view of the tip of the piston of Figure 10A;
Figure 11 is a side elevational view of the tissue homogenizing device of Figure 10A as configured for automated use in still a further stage of use thereof;
Figure 12 is a side cross sectional elevational view of a universal tissue homogenizing device, in accordance with still another illustrative embodiment of the present disclosure, illustrating a stage of use thereof;
Figure 13 is a side elevational view of the tissue homogenizing device of Figure 12 illustrating another stage of use thereof; and
Figure 14 is a perspective view of a distal end of the tissue homogenizing device of Figures 12 and 13.

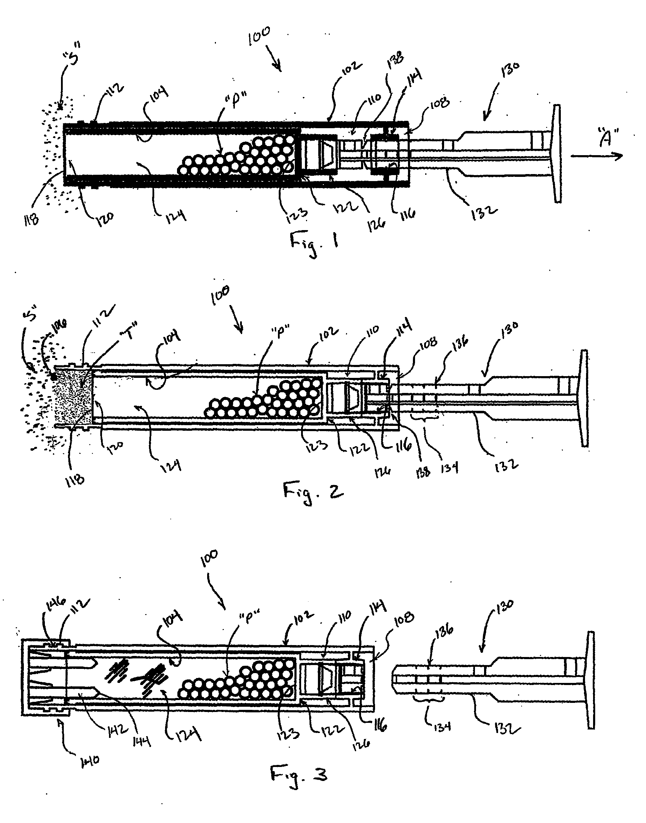

Preferred embodiments of the presently disclosed tissue homogenizer will now be described more fully hereinafter with reference to the accompanying drawings. Referring to FIGS. 1-5, a universal tissue homogenizing device, in accordance with one illustrative embodiment of the present disclosure, is generally designated as 100. Although the presently disclosed tissue homogenizing device 100 will be described and illustrated hereinafter in connection with specific embodiments and uses, such as, for example, use in the medical field, it will be readily appreciated and understood by one skilled in the art that the presently disclosed tissue homogenizing device 100 may be adapted for usage in other applications and fields of use as well.

In the drawings and in the description that follows, the term "proximal", as is traditional, will refer to the end of the instrument, device and/or apparatus which is closest to the operator while the term "distal" will refer to the end of the instrument, device and/or apparatus which is furthest away from the operator.

As seen in FIGS. 1-5, tissue homogenizing device 100 includes a pair of concentric cylinders, namely an outer cylinder 102 and an inner cylinder 104, wherein outer and inner cylinders 102 and 104 are longitudinally displaceable relative to one another. Outer cylinder 102 includes an open distal end 106 and an open proximal end 108 defining a lumen 110 therebetween. Preferably, at least one engaging member 112 (e.g., in the form of helical threads, bayonet-type structure, etc.) is provided on the outer surface of outer cylinder 102 at or near distal end 106. Outer cylinder 102 includes a hub 114 operatively connected to and/or integrally formed with proximal end 108. Hub 114 defines an opening 116 axially aligned with the central axis of outer cylinder 102.

Inner cylinder 104 defines a chamber 124 including an open distal end 120 and a closed proximal end 122 defined by a proximal end wall 123. Inner cylinder 104 further includes a hub 126 operatively connected to and/or integrally formed on an outer surface of proximal end wall 123. Hub 126 of inner cylinder 104 is preferably axially aligned with hub 114 of outer cylinder 102. A seal 118 is preferably disposed across distal end 120 to effectively cap and/or close distal end 120 of inner cylinder 104. In this manner, chamber 124 of inner cylinder 104 can be hermetically sealed against contamination from the outside environment until device 100 is used. Preferably, inner cylinder 104 has an overall length that is less than the overall length of outer cylinder 102.

Tissue homogenizing device 100 further includes an amount of particulate matter "P" including and not limited to pellets, granules, shots, "BBs", aggregate and the like contained in chamber 124 of inner cylinder 104. Chamber 124 can also contain various reagents that facilitate assaying of a sample, such as, for example, buffers, preservatives, solvents, specific binding proteins (e.g. antibodies, antigens, peptides) and marking reagents.

Tissue homogenizing device 100 further includes a plunger and/or piston 130 having a piston rod 132 configured and dimensioned to extend through opening 116 of hub 114 provided in outer cylinder 102 and configured and dimensioned to operatively engage hub 126 of inner cylinder 104. Accordingly, as will be described in greater detail below, in use, as piston 130 is displaced in an axially proximal and/or distal direction relative to outer cylinder 102, inner cylinder 104 is also displaced in a corresponding axially proximal and/or distal direction.

Preferably, piston rod 132 of piston 130 includes at least one, and more preferably, a plurality of marks 134 (e.g., grooves, ticks, marks, indicia or the like) formed thereon at a distal end 136 thereof which indicate to the operator the distance piston 130 has been displaced relative to outer cylinder 102. In addition, as will be described in greater detail below, each mark 134 indicates to the operator the amount of sample "S" drawn into distal end 106 of outer cylinder 102.

Piston rod 132 of piston 130 further includes a region of reduced strength 138 (i.e., a break-away area) wherein a portion of piston 130, proximal of region 138, can be broken away from the portion of piston 130 distal of region 138. Alternatively, a distal end of piston rod 132 can include an inter-engaging structure (not shown), e.g., helical threads, for engaging hub 126 of inner cylinder 104 such that piston rod 132 can be removably attached to hub 126 of inner cylinder 104.

With continued reference to FIGS. 1-3, a method of use of tissue homogenizing device 100 will now be described. In use, with piston 130 in a distal position such that distal end 106 of inner cylinder 104 is substantially flush with distal end 106 of outer cylinder portion 102, distal end of outer cylinder 102 is inserted into a quantity of sample "S". Preferably, distal end 106 of inner cylinder 104 is flush with distal end 106 of outer cylinder 102. As seen in FIG. 2, with distal end 106 disposed in the quantity of sample "S", piston 130 is withdrawn in a proximal direction (as indicated by arrow "A") relative to outer cylinder 102 thereby displacing inner cylinder 104 in a proximal direction relative to outer cylinder 102. Accordingly, a vacuum is formed at the distal end thereof and a test sample "T" of the quantity of sample "S" is drawn into distal end 106 of outer cylinder 102.

Preferably, piston 130 is displaced, in a proximal direction (as indicated by arrow "A"), a distance sufficient to draw in a desired amount of the quantity of sample "S" into distal end 106 of outer cylinder 102. As mentioned above, the quantity of sample "S" drawn into distal end 106 of outer cylinder 102 directly corresponds to the number of marks 134 of piston 130 exposed from outer cylinder 102 when piston 130 is displaced in the proximal direction. For example, marks 134 can be spaced from one another and outer cylinder 102 can be dimensioned such that each mark 134 represents a fixed volume, such as, for example, 100µl of the quantity of sample "S" being drawn into distal end 106 of outer cylinder 102. In this exemplary embodiment, outer cylinder 102, inner cylinder 104 and markings 134 of piston 130 are configured and dimensioned such that when piston 130 is displaced a maximum amount in the proximal direction hub 126 of inner cylinder 104 contacts hub 114 of outer cylinder 102, three marks 134 are exposed and 300µL of the quantity of sample "S" is drawn into distal end 106 of outer cylinder 102. Sample acquisition can be facilitated by rotation or other manipulation by the user. For example, when the sample is disposed adjacent a relatively rigid surface, device 100 can be manipulated in a manner similar to operating a "cookie-cutter".

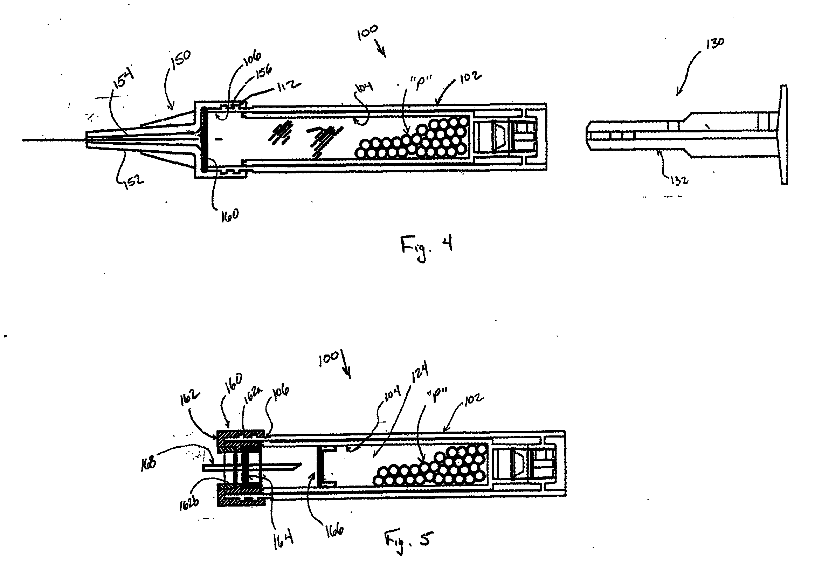

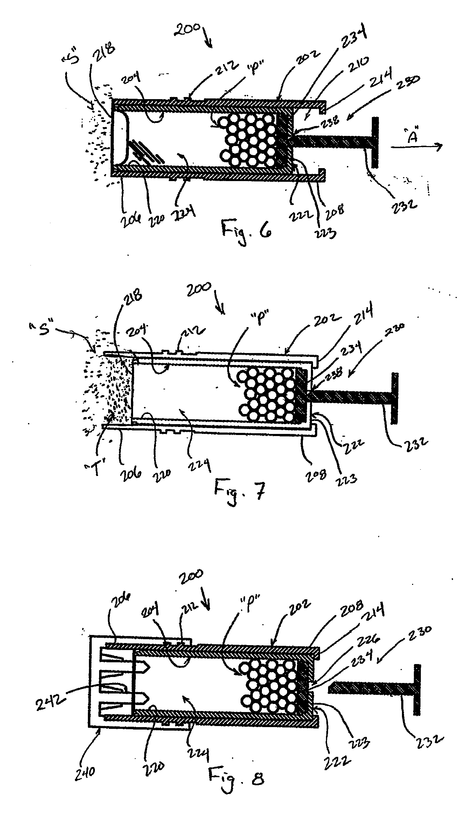

As seen in FIG. 3, with test sample "T" drawn into distal end 106 of outer cylinder 102, a closure cap 140, configured and dimensioned to removably engage distal end 106 of outer cylinder 102, is coupled to distal end 106 of outer cylinder 102. Cap 140 includes at least one, preferably a plurality of, penetrating members such as rods 142 extending longitudinally from an inner surface thereof. Each rod 142 preferably includes a tip 144 (e.g., in the form of a sharpened point, taper, cone and the like) configured and dimensioned to penetrate seal 118. Accordingly, when cap 140 is coupled to distal end 106 of cylinder 102, rods 142 penetrate seal 118 thereby allowing test sample "T" to enter chamber 124 of inner cylinder 104 and combine and/or mix with particulate matter "P" and any reagents contained therein. Preferably, cap 140 includes at least one engaging member 146 configured and adapted to inter-engage with engaging member 112 provided on the outer surface of outer cylinder 102. In this manner, cap 140 is prevented from inadvertently becoming separated and/or otherwise disassociated from outer cylinder 102.

With cap 140 secured on distal end 106 of outer cylinder 102, the portion of piston 130 proximal of region 138 is broken-off and/or otherwise separated from the portion of piston 130 distal of region 138, preferably along region 138.

With test sample "T" now contained in chamber 124, tissue homogenizing device 100 can be placed in an agitating apparatus, a homogenizer and/or the like, such as a ribolyser (e.g. a FastPrep® ribolyser available from Bio 101, Inc., or a Magnalyser™ available from Roche) whereby particulate matter "P" acts to agitate, grind or otherwise break-up test sample "T".

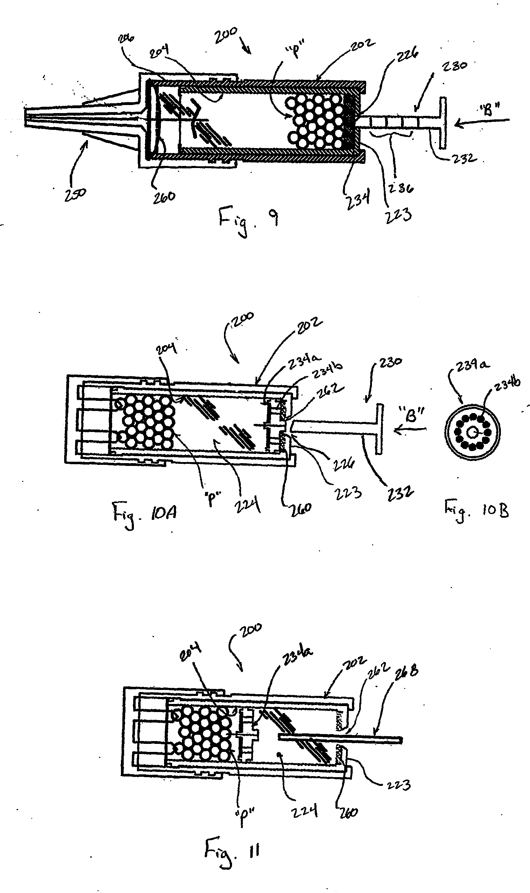

Following homogenization of test sample "T", tissue homogenizing device 100 can be configured for either manual and/or robotic use. As seen in FIG. 4, tissue homogenizing device 100 is configured for manual use by removing cap 140 and attaching a dispensing cap 150 to distal end 106 of outer cylinder 102. Dispensing cap 150 is funnel-like including a frusto-conical tip 152 defining a lumen 154 therethrough. Preferably, a filter 157 is disposed between dispensing cap 150 and distal end 106 of outer cylinder 102. Filter 160 functions to strain out particulate matter "P" from test sample "T". Dispensing cap 150 can include at least one engaging member 156 configured and adapted to inter-engage with engaging member 112 provided on the outer surface of outer cylinder 102. In this manner, dispensing cap 150 is prevented from inadvertently becoming separated and/or otherwise disassociated from outer cylinder 102.

Dispensing cap 150 may further include a stem (not shown) extending proximally of tip 152. The stem may be configured and dimensioned to be received within the distal end of inner cylinder 104. Preferably, the stem may form a fluid tight seal with the inner surface of inner cylinder 104. The stem may include a lumen (not shown) formed therein which is in fluid communication with lumen 154 of tip 152. The lumen of the stem may be separated from lumen 154 by filter 157. The stem provides volume displacement of air (i.e., the stem replaces the dead volume created by cap 140 that tears the seal at the distal end of chamber 124). The stem is useful in that there is a limited amount of stroke available to inner cylinder 104 relative to outer cylinder 102 and thus the air would interfere with and/or otherwise inhibit the user's ability to discharge a desired volume of sample "S".

With dispensing cap 150 connected to distal end 106 of outer cylinder 102, the distal end of piston rod 132 is re-introduced into hub 114 of outer cylinder 102 to operatively engage hub 126 of inner cylinder 104. As such, piston 130 may be advanced in a distal direction to push sample "S" out through lumen 154 of dispensing cap 150. As is envisioned, inner cylinder 104 has been displaced in a distal direction relative to outer cylinder 102 and sample "S" has been forced through the lumen of the stem and not along the outer surface of stem 158.

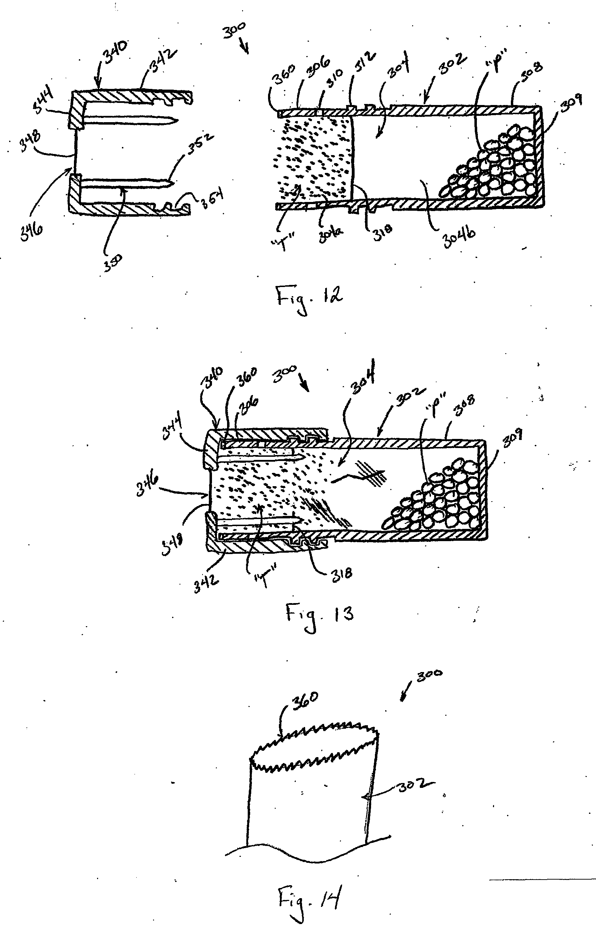

Alternatively, as seen in FIG. 5, tissue homogenizing device 100 can be configured for robotic use by attaching a septum cap 160 to distal end 106 of outer cylinder 102. Septum cap 160 includes an annular wall 162 having a substantially U-shaped cross-sectional profile defined by an outer wall 162a and an inner wall 162b. Septum cap 160 includes a septum seal 164, preferably rubber, supported on inner wall 162b. Prior to attaching septum cap 160 to outer cylinder 102, a filter 166 is desirably slidingly positioned within chamber 124 of inner cylinder 104. In this manner, filter 166 can be moved axially along chamber 124 as needed and/or desired. In use, a tip of a septum piercing pipette 168 penetrates septum seal 164 and it used to withdraw a quantity of test sample "T" from chamber 124. Since filter 166 is slidingly positioned in chamber 124, filter 166 can be repositioned as needed to avoid being penetrated by the tip of pipette 168, as seen in FIG. 5.

Turning now to FIGS. 6-11, a tissue homogenizing device, in accordance with yet another illustrative embodiment of the present disclosure, is shown generally as 200. As seen in FIGS. 6-8, tissue homogenizing device 200 includes a pair of concentric cylinders, namely an outer cylinder 202 and an inner cylinder 204, wherein outer and inner cylinders 202 and 204 are longitudinally displaceable relative to one another. Outer cylinder 202 includes an open distal end 206 and an open proximal end 208 defining a lumen 210 therebetween. Preferably, at least one engaging member 212 is provided on the outer surface of outer cylinder 202. Proximal end 208 of outer cylinder 202 includes a radially inward extending annular flange and/or rim 214.

Inner cylinder 204 defines a chamber 224 including an open distal end 220 and a closed proximal end 222 defined by a proximal end wall 223. Inner cylinder 204 further includes an opening 226 formed in proximal end wall 223. A seal 218 is preferably disposed across distal end 220 to effectively cap and/or close distal end 220 of inner cylinder 204. In this manner, chamber 224 of inner cylinder 204 can be hermetically sealed against contamination from the outside environment until device 200. Preferably, inner cylinder 204 has an overall length which is less than the overall length of outer cylinder 202.

As described above with respect to tissue homogenizing device 100, tissue homogenizing device 200 further includes an amount of particulate matter "P" contained in chamber 224 of inner cylinder 204.

Tissue homogenizing device 200 further includes a plunger and/or piston 230 having piston rod 232 slidably extending through proximal end 208 of outer cylinder 202 and through opening 226 formed in proximal end wall 223 of inner cylinder 204. Piston 230 includes a head 234 operatively coupled to and/or integrally formed with a distal end of piston rod 232. Preferably, head 234 is sized and dimensioned to contact the inner surface of inner cylinder 204 along the entire periphery thereof. Piston rod 232 includes at least one, and more preferably, a plurality of marks 236 (see FIG. 9) formed thereon which indicate to the operator the distance piston 230 has been displaced relative to outer cylinder 202.

Piston rod 232 further includes a region of reduced strength 238 wherein a portion of piston 230, proximal of head 234, can be broken away therefrom, preferably along region 238. Alternatively, a distal end of piston rod 232 can include an inter-engaging structure (not shown), e.g., helical threads, while a proximal surface of head 234 can include a complementary inter-engaging structure (not shown), e.g., a helically threaded bore, such that piston rod 232 can be removably attached to head 234.

With continued reference to FIGS. 6-8, a method of use of tissue homogenizing device 200 will now be described. In use, with distal end 206 of outer cylinder 202 substantially flush with distal end 220 of inner cylinder 204 and with piston 230 positioned such that head 234 rests against and/or is in contact with proximal end wall 223 of inner cylinder 204, distal end of outer cylinder 202 is inserted into a quantity of sample "S". As seen in FIG. 7, with distal end 206 of outer cylinder 202 disposed in the quantity of sample "S", piston 230 is withdrawn in a proximal direction (as indicated by arrow "A") relative to outer cylinder 202 thereby displacing inner cylinder 204 in a proximal direction relative to outer cylinder 202. Accordingly, a vacuum is formed at the distal end thereof and a test sample "T" of the quantity of sample "S" is drawn into distal end 206 of outer cylinder 202.

Preferably, piston 230 is displaced, in a proximal direction, a distance sufficient to draw in a desired amount of the quantity of sample "S" into distal end 206 of outer cylinder 202. Marks 236 of piston 230 function in the same manner as marks 134 of piston 130 described above. The maximum distance inner cylinder 204 can be proximally displaced relative to outer cylinder 202 is defined by annular flange 214 which acts as a stop to the displacement of inner cylinder 204 relative to outer cylinder 202.

As seen in FIG. 8, with test sample "T" drawn into distal end 206 of outer cylinder 202, a closure cap 240, similar to closure cap 140 described above, is coupled to distal end 206 of outer cylinder 202. Closure cap 240 includes a plurality of rods 242 configured and dimensioned to penetrate seal 218. Accordingly, when closure cap 240 is coupled to distal end 206 of outer cylinder 202, rods 242 penetrate seal 218 thereby allowing test sample "T" to enter chamber 224 of inner cylinder 204 and combine and/or mix with particulate matter "P" contained therein.

With closure cap 240 secured on distal end 206 of outer cylinder 202, the portion of piston 230 proximal of head 234 is broken-off and/or otherwise separated therefrom, preferably along region 236.

As with tissue homogenizing device 100, with test sample "T" now contained in chamber 224 and combined with particulate matter "P", tissue homogenizing device 200 can be placed in an agitating apparatus, a homogenizer, ribolyser, or the like whereby particulate matter "P" acts to agitate and/or break-up test sample "T".

Following separation and/or homogenization of test sample "T" tissue homogenizing device 200 can be configured for either manual and/or robotic use. As seen in FIG. 9, tissue homogenizing device 200 is configured for manual use by removing closure cap 240 and attaching a dispensing cap 250, similar to dispensing cap 150 described above, to distal end 206 of outer cylinder 202. Preferably, a filter 260 is disposed between dispensing cap 250 and distal end 206 of outer cylinder 202.

With dispensing cap 250 and filter 260 operatively associated with distal end 206 of outer cylinder 202, inserting a distal end of piston rod 232 into opening 226 formed in proximal end wall 223 of inner cylinder 204 such that the distal end of piston rod 232 engages head 234. With piston rod 232 engaged with head 234, piston 230 is displaced in a distal direction (as indicated by arrow "B") an amount sufficient to expel and/or otherwise eject test sample "T" from tissue homogenizing device 200.

Alternatively, as seen in FIGS. 10A, 10B and 11, tissue homogenizing device 200 can be configured for robotic use. As so configured, prior to use, head 234 of piston 230 is replaced with a filter head 234a including a plurality of apertures 234b formed therethrough which act to filter out particulate and the like. Preferably, apertures 234b are arranged in an annular array therearound. A septum seal 260 is preferably disposed between filter head 234a and proximal end wall 223 of inner cylinder 204 and includes an opening 262 formed therein.

Accordingly, following separation and/or homogenization of test sample "T", piston rod 232 is inserted through opening 226 formed in proximal end wall 223, through opening 262 formed in septum seal 260, and attached to and/or otherwise engaged with filter head 234a and displaced in a distal direction (as indicated by arrow "B") to thereby displace filter cap 234a in a distal direction through chamber 224 and separate particulate matter "P" from test sample "T". With test sample "T" filtered and filter cap 234a repositioned, a tip of a pipette 268 is inserted into chamber 224 through opening 262 of septum seal 260. Pipette 268 is then used to withdraw a quantity of test sample "T" from chamber 224.

Turning now to FIGS. 12 and 13, a tissue homogenizing device, in accordance with yet another illustrative embodiment of the present disclosure, is shown generally as 300. As seen in FIGS. 12 and 13, tissue homogenizing device 300 includes a cylindrical container 302 having an open distal end 306 and a closed proximal end 308 defined by a proximal end wall 309. Container 302 defines a chamber 304 therein. Container 302 preferably includes at least one engaging member 312 (e.g., in the form of helical threads, bayonet-type structure, etc.) provided on the outer surface thereof at or near distal end 306.

Preferably, container 302 includes at least one aperture and/or vent hole 310 formed at or near distal end 306, preferably at a location distal of engaging member 312. A seal 318 is disposed across chamber 304 to thereby divide chamber 304 into a distal reservoir 304a and a proximal reservoir 304b. Preferably, seal 318 is disposed at a location proximal of vent hole(s) 310. In this manner, as will be described in greater detail below, vent hole(s) 310 allow displacement of air from distal reservoir 304a when a quantity of sample "S" is introduced into distal reservoir 304a. Preferably, seal 318 is positioned within chamber 304 at a location which fixes and/or defines the volume of distal reservoir 304a to a predetermined amount.

As with the previously described embodiments, tissue homogenizing device 300 also further includes an amount of particulate matter "P" contained in proximal reservoir 304b of chamber 304.

Tissue homogenizing device 300 further includes a closure cap 340, configured and dimensioned to removably engage distal end 306 of container 302. Cap 340 includes an annular side wall 342 supporting an end wall 344 on a distal surface thereof. End wall 344 includes an opening 346 formed therethrough and a septum seal 348 (e.g., a rubber septum seal) extending across opening 346. Side wall 342 preferably has a length sufficient to cover and/or otherwise block vent hole(s) 310 and thereby prevent the escape of sample "S" from container 302.

Cap 340 further includes at least one, preferably a plurality of, penetrating members such as rods 350 extending longitudinally from an inner surface of end wall 344. Each rod 350 preferably includes a tip 352 (e.g., in the form of a sharpened point, taper, cone and the like) configured and dimensioned to penetrate seal 318 when cap 340 is coupled to distal end 306 of container 302. Cap 340 further includes at least one engaging member 354 extending from an inner surface of side wall 342. Engaging member(s) 354 is/are configured and dimensioned to inter-engage with engaging member 312. As such, cap 340 is prevented from inadvertently becoming separated and/or otherwise disassociated from container 302.

Preferably, as seen in FIG. 14, distal end 306 of container 302 is provided with a cutting edge 360. Preferably, cutting edge 360 is serrated. Cutting edge 360 acts like and can be used by the user of tissue homogenizing device 300 as a knife or similar cutting instrument/device, to facilitate in the cutting and/or acquisition of tissue.

With continued reference to FIGS. 12 and 13, a method of use of tissue homogenizing device 300 will now be described. In use, a user (e.g., surgeon, nurse, technician or the like) places a quantity of sample "S" into distal reservoir 304a by scooping, pressing and/or otherwise transferring sample "S" to distal reservoir 304a. Vent hole(s) 310, as described above, allow for the escape of air from distal reservoir 304a and thus enable greater quantities of sample "S" to be transferred to distal reservoir 304a. Preferably, vent hole(s) 310 allow the entire volume of distal reservoir 304a to be filled with sample "S". By filling distal reservoir 304a to capacity, a uniform amount of sample "S" can be consistently gathered and thereafter processed.

With a quantity of sample "S" placed in distal reservoir 304a or, preferably, with distal reservoir 304a filled with sample "S", closure cap 340 is coupled to distal end 306 of container 302. In so doing, rods 350 penetrate seal 318 thereby allowing test sample "T" to enter proximal reservoir 304b and combine and/or interact with particulate matter "P" contained therein.

With test sample "T" contained within chamber 304 and with cap 350 secured on distal end 306 of container 302, tissue homogenizing device 300 can be placed in an agitating apparatus, a homogenizer and/or the like whereby particulate matter "P" acts to agitate and/or break-up test sample "T".

Following separation and/or homogenization of test sample "T", a tip of a septum piercing pipette 168 (see FIG. 5) can be used to penetrate septum seal 348 of cap 340 and is used to withdraw a quantity of test sample "T" from chamber 304.

It is envisioned that a hypodermic needle assembly (not shown) can be removably attached to the distal end of the outer cylinder of any of the above-described devices to facilitate collection of test sample "T" into the distal end of the outer cylinder.

It is envisioned that tissue homogenizing devices 100, 200, and 300 can be used in connection with sample acquisition and/or distribution procedures wherein homogenization of the sample is desires and/or required, such as, for example, in the testing for Bovine Spongiform Encephalopathy, otherwise known as "Mad Cow" disease.

Although the present disclosure has been described with respect to preferred embodiments, it will be readily apparent to those having ordinary skill in the art to which it appertains that changes and modifications may be made thereto without departing from the spirit or scope of the disclosure.

## Claims

1. A tissue homogenizer device for testing a sample of blood and/or tissue, the device comprising an outer cylinder including an open distal end, an open proximal end, and defining a lumen therethrough an inner cylinder slidably disposed within the lumen of the outer cylinder, the inner cylinder including an open distal end, a closed proximal end, and defining a chamber therewithin a seal extending across the open distal end of the inner cylinder, and a piston including a piston rod extending proximally from the closed proximal end of the inner cylinder and through the open proximal end of the outer cylinder, wherein axial displacement of the piston axially displaces the inner cylinder relative to the outer cylinder, the device including a first position in which the distal end of the inner cylinder is in close proximity to the open distal end of the outer cylinder, and a second position in which the distal end of the inner cylinder is spaced a distance from the open distal end of the outer cylinder.

2. The device according to claim 1, wherein when the device is in the first position the proximal end of the inner cylinder is spaced a distance from the proximal end of the outer cylinder, and when the device is in the second position the proximal end of the inner cylinder is in close proximity with the proximal end of the outer cylinder.

3. The device according to claim 1, further including particulate in the chamber of the inner cylinder.

4. The device according to claim 3, wherein the particulate includes at least one of pellets, granules, shots, BBs and aggregate.

5. The device according to claim 3, wherein the piston rod includes a region of reduced strength formed along the length thereof.

6. The device according to claim 5, further including a cap removably connectable to the open distal end of the outer cylinder.

7. The device according to claim 6, wherein the cap includes at least one rod extending therefrom for penetrating the seal of the inner cylinder when the cap is secured to the distal end of the outer cylinder.

8. The device according to claim 7, wherein the cap includes an engagement element for securing the cap to a complementary engagement element provided at the distal end of the outer cylinder.

9. The device according to claim 8, further including a dispensing cap removably connectable to the open distal end of the outer cylinder, wherein the dispensing cap includes a lumen extending therethrough.

10. The device according to claim 9, wherein the dispensing cap includes an engagement element for securing the cap to a complementary engagement element provided at the distal end of the outer cylinder.

11. The device according to claim 10, wherein the dispensing cap includes a filter extending across the lumen thereof.

12. The device according to claim 8, further comprising a septum cap removably connectable to the open distal end of the outer cylinder, the septum cap including an annular outer wall, an annular inner wall defining a passage and a septum seal extending across the passage.

13. The device according to claim 12, further comprising a filter slidably disposed in the chamber of the inner cylinder.

14. The device according to claim 1, wherein the proximal end of the outer cylinder includes a hub operatively associated therewith through which the piston rod extends, and the proximal end of the inner cylinder includes a hub operatively associated therewith to which the piston rod is secured.

15. The device according to claim 11, wherein the piston rod slidably extends through an opening formed in the closed proximal end of the inner cylinder, and wherein the piston includes a head operatively connected to a distal end of the piston rod.

16. The device according to claim 15, wherein the head includes at least one aperture formed therethrough.

17. The device according to claim 16, wherein the piston rod includes a region of reduced strength formed along the length thereof enabling the piston rod to be separated from the head.

18. The device according to claim 17, wherein the piston rod reciprocatingly drives the head axially through the chamber of the inner cylinder.

19. The device according to claim 18, further comprising a filter extending across the opening formed in the proximal end of the inner cylinder.

20. The device according to claim 1, wherein the open proximal end of the outer cylinder includes a flange formed therearound.

21. The device according to claim 1, wherein the piston rod includes a series of indicia along the length thereof, wherein the indicia at least one of indicates a quantity of a sample drawn into the chamber and indicates a quantity of a sample expressed from the chamber.

22. A tissue homogenizer device for testing a sample of blood and/or tissue, the device comprising a cylinder including an open distal end, a closed proximal end, and defining a chamber therewithin a seal extending across the chamber to define a closed proximal reservoir and an open distal reservoir, a particulate disposed in the proximal reservoir, and a cap operatively securable to the distal end of the cylinder, the cap including an end wall defining an opening therein a seal extending across the opening formed in the end wall of the cap, and at least one puncturing element extending from an inner surface of the end wall of the cap, wherein the puncturing element is configured to penetrate the seal when the cap is secured to the distal end of the cylinder.

23. The device according to claim 22, wherein the distal end of the cylinder includes a cutting edge.

24. The device according to claim 23, wherein the cutting edge is serrated.

25. The device according to claim 23, wherein the cap includes an engagement element for engaging a complementary engagement element provided on the cylinder.

26. A method of manipulating a tissue or blood sample, comprising the steps of providing a tissue homogenizing device, the device including an outer cylinder having an open distal end, an open proximal end, and defining a lumen therethrough, an inner cylinder slidably disposed within the outer cylinder, the inner cylinder having an open distal end, a closed proximal end, and defining a chamber therewithin a seal extending across the chamber of the inner cylinder, a particulate disposed within the chamber of the inner cylinder, a piston having a piston rod extending through the open proximal end of the outer cylinder and operatively associated with the closed proximal end of the inner cylinder, and a cap securable to the distal end of the outer cylinder, the device has a first position in which the proximal end of the inner cylinder is spaced a distance from the proximal end of the outer cylinder, wherein the distal end of the inner cylinder does not extend beyond the distal end of the outer cylinder, and at least one second position in which the proximal end of the inner cylinder is in close proximity to the proximal end of the outer cylinder inserting the distal ends of the outer cylinder and the inner cylinder into a quantity of a sample at least one of withdrawing the piston to proximally displace the inner cylinder relative to the outer cylinder to evacuate the lumen of the outer cylinder and draw in a test sample of the quantity of sample, and urging at least the distal end of the outer cylinder into the quantity of the sample, and securing the cap onto the distal end of the outer cylinder.

27. The method according to claim 26, wherein the cap includes a piercing element extending therefrom such that when the cap is secured to the distal end of the outer cylinder the piercing element penetrates the seal.

28. The method according to claim 27, further comprising the step of separating the piston from the inner cylinder.

29. The method according to claim 28, wherein the piston rod includes a series of indicia along the length thereof indicating a volume of test sample acquired in the lumen of the outer cylinder.

30. The method according to claim 29, further comprising the step of agitating the device following separation of the piston from the inner cylinder.

31. The method according to claim 30, further comprising the step of replacing the cap with a dispensing cap following agitation of the device.

32. The method according to claim 31, wherein the dispensing cap defines a lumen extending therethrough and includes a filter extending across the lumen thereof.

33. The method according to claim 32, further comprising the step of urging the inner cylinder in a distal direction relative to the outer cylinder to force the test sample through the filter and out through the lumen of the dispensing cap.

34. The method according to claim 32, wherein the piston rod slidably extends through an opening formed in the proximal end of the inner cylinder, and wherein the piston includes a head provided on the distal end of the piston rod and disposed within the chamber of the inner cylinder.

35. The method according to claim 34, wherein the piston rod has a first position in which the head is in close proximity to the proximal end of the inner cylinder and at least one second position in which the head is spaced a distance from the proximal end of the inner cylinder.

36. The method according to claim 35, wherein the piston rod is separable from the head.

37. The method according to claim 36, wherein the device further comprises a septum seal extending across the opening formed in the proximal end of the inner cylinder.

38. The method according to claim 37, further comprising the step of urging the head from the first position to at least one second position.

39. The method according to claim 38, further comprising the step of inserting a pipette into the chamber of the inner cylinder through the opening in the proximal end of the inner cylinder.

40. The method according to claim 39, further comprising the step of withdrawing the test sample from the chamber of the inner cylinder.

41. The method according to claim 35, wherein the head includes at least one aperture formed therethrough.

42. The method according to claim 30, further comprising the step of replacing the cap with a septum cap, wherein the septum cap defines a passage therethrough and which includes a seal extending across the passage.

43. The method according to claim 42, wherein the device further comprises a filter slidably disposed within the chamber of the inner cylinder.

44. A method of manipulating a tissue sample, comprising the steps of providing a tissue homogenizing device, the device including at least one cylinder having a distal end, a proximal end and a lumen defined therethrough, a fluid-tight seal member associated with the at least one cylinder adapted for longitudinal movement therein, and particulate material disposed within the at least one cylinder, disposing a tissue sample within the at least one cylinder, imparting mechanical energy to the at least one cylinder to cause the particulate material to interact with the tissue sample in a manner that breaks up the tissue sample, and manipulating the fluid-tight seal member to dispense a portion of the tissue sample from the at least one cylinder.

45. The method according to claim 44, further comprising the step of providing a predetermined amount of a reagent useful for conducting an immunoassay and disposing said reagent in the at least one cylinder.
